# EUROPEAN PATENT APPLICATION

(11) **EP 4 194 004 A1**
(43) Date of publication of application: **14.06.2023**
(21) Application number: 21833677.4
(22) Date of filing: 01.03.2021
(51) Int. Cl.: A61K 38/22, A61P 31/14, A61P 31/16, A61P 37/06, A61P 43/00

(54) **STAT3 PHOSPHORYLATION INHIBITOR, AND PROPHYLACTIC OR THERAPEUTIC AGENT FOR AUTOIMMUNE DISEASE AND CORONAVIRUS INFECTION**

(30) Priority: 30.06.2020 JP 2020112680
(71) Applicant: Tohoku University, Sendai-shi, Miyagi 980-8577 (JP)
(72) Inventor: OHKOUCHI, Shinya, Sendai-shi, Miyagi 980-8577 (JP); KANEHIRA, Masahiko, Sendai-shi, Miyagi 980-8577 (JP); OKADA, Yoshinori, Sendai-shi, Miyagi 980-8577 (JP); KUROSAWA, Hajime, Sendai-shi, Miyagi 980-8577 (JP)
(74) Representative: Winter, Brandl - Partnerschaft mbB
(86) International application number: PCT/JP2021/007780
(87) International publication number: WO 2022/004054

(57) **Abstract**

A STAT3 phosphorylation inhibitor containing stanniocalcin 1 (STC1) as an active ingredient. A JAK expression or phosphorylation inhibitor containing stanniocalcin 1 as an active ingredient. A prophylactic or therapeutic agent for autoimmune disease, the agent containing stanniocalcin 1 as an active ingredient. A prophylactic or therapeutic agent for influenza virus infection, the agent containing stanniocalcin 1 as an active ingredient. A prophylactic or therapeutic agent for coronavirus infection, the agent containing stanniocalcin 1 as an active ingredient.

## Description

### TECHNICAL FIELD

### [Cross Reference of Related Application]

The present application claims based on JP 2020-112680 filed on June 30, 2020 (the entire disclosure of which is incorporated in the present specification by reference). The present invention relates to a prophylactic or therapeutic agent for autoimmune disease and coronavirus infection.

### BACKGROUND ART

In the respiratory field, the inhibitory effect of mesenchymal stem cells (MSCs) on lung injury/fibrogenesis and anti-inflammatory effect have received attention (Non-Patent Literature 3). In addition, inflammatory cytokines, such as TNF-α, IL-1β, CCL2, and IL-6, are important factors in cytokine storm. In particular, a mechanism of inducing IL-6 through the IL-6/JAK/STAT transduction pathway or the like is called IL-6 amplifier (IL-6 AMP) and serves as a major mechanism of cytokine storm, which is considered to be the cause of aggravation of autoimmune disease represented by rheumatoid arthritis, and COVID-19 and the like (Non-Patent Literature 4).

Autoimmune disease is a general term for diseases due to breakdown of immune tolerance, where the immune system overreacts against and attacks normal cells and/or tissues of oneself, thus causing a symptom. Many diseases are classified as autoimmune diseases including those designated as intractable diseases, thus leading to a demand for development of the therapeutic method.

In addition, although coronaviruses are known as viruses causing symptoms of what is called a cold, mutation may allow highly infectious and toxic strains to arise, which has been problematic. In particular, coronavirus disease 2019 (COVID-19), what is called new coronavirus infection, which is an infectious disease of severe acute respiratory syndrome coronavirus 2 (SARS-CoV2), a mutant as described above, is spreading all over the world and causing many deaths, and thus there is an urgent need to develop the therapeutic method.

### CITATION LIST

### Patent Literature

Patent Literature 1: JP 5946191 B

### Non-Patent Literature

Non-Patent Literature 1: Hoffmann et al. Cell 181, 1-10 (2020)
Non-Patent Literature 2: Website of the University of Tokyo, "Shingata Korona Uirusu no Kansen-Soshi ga Kitai-Sareru Kokunai Kizon Yakuzai no Dotei (Identification of Existing Drugs in Japan Expected to Prevent Infection with New Coronavirus)": https://www.ims.u-tokyo.ac.jp/imsut/content/000002328.pdf
Non-Patent Literature 3: Clin Med Insights Circ Respir Pulm Med. 2015; 9: 91-6
Non-Patent Literature 4: Immunity. 2020; 52: 731-33.
Non-Patent Literature 5: Mol Immunol 2007 2497
Non-Patent Literature 6: UIRUS (Virus), Vol. 69, No. 1, pp. 61-72, 2019

### SUMMARY OF INVENTION

### Technical Problem

An object to be solved by the present invention is to provide a novel STAT3 phosphorylation and IL6 AMP inhibitor. In addition, another object of the present invention is to provide a novel prophylactic or therapeutic agent for a disease that can be treated by inhibiting STAT3 phosphorylation. For example, a further object to be solved by the present invention is to provide a novel prophylactic or therapeutic agent for autoimmune disease, the agent containing a substance that has not been known for its therapeutic effect on autoimmune disease as an active ingredient. Furthermore, yet another object of the present invention is to provide a novel prophylactic or therapeutic agent for coronavirus infection, the agent containing a substance that has not been known for its therapeutic effect on coronavirus infection as an active ingredient.

### Solution to Problem

Under such circumstances, as a result of study of several thousands of compounds, the present inventors have found that stanniocalcin 1 (STC1) inhibits the phosphorylation of STAT3 located downstream of the IL-6/JAK/STAT transduction pathway. Furthermore, the present inventors used STC1 and obtained the following findings about STAT3 phosphorylation. Specifically, the present inventors have also found that STC1 epigenetically induces the expression of anti-inflammatory factor SOCS1. Moreover, the present inventors have also found that STC1 inhibits the expression of factors important for the receptor for the s protein of a coronavirus, the receptor located on the cell surface, and fusion of the viral outer membrane and the cell membrane, and thus inhibits infection of the cell with the coronavirus. The present invention is based on these novel findings.

Thus, the present invention provides the following items:
Item 1. A STAT3 phosphorylation inhibitor containing stanniocalcin 1 (STC1) as an active ingredient.
Item 2. A JAK expression or phosphorylation inhibitor containing stanniocalcin 1 as an active ingredient.
Item 3. A prophylactic or therapeutic agent for autoimmune disease, the agent containing stanniocalcin 1 as an active ingredient.
Item 4. A prophylactic or therapeutic agent for influenza virus infection, the agent containing stanniocalcin 1 as an active ingredient.
Item 5. A prophylactic or therapeutic agent for coronavirus infection, the agent containing stanniocalcin 1 as an active ingredient.
Item 6. The prophylactic or therapeutic agent for coronavirus infection according to item 5, wherein the coronavirus has an S protein capable of binding to ACE2.
Item 7. The prophylactic or therapeutic agent for coronavirus infection according to item 5 or 6, wherein the coronavirus is a SARS-related coronavirus.
Item 8. An ACE2 and TMPRSS2 expression inhibitor containing stanniocalcin 1 as an active ingredient.
Item 9. An IL6 AMP inhibitor containing stanniocalcin 1 as an active ingredient.
Item 10. The STAT3 phosphorylation inhibitor according to item 1, the JAK expression or phosphorylation inhibitor according to item 2, the prophylactic or therapeutic agent for autoimmune disease according to item 3, the prophylactic or therapeutic agent for influenza virus infection according to item 4, the prophylactic or therapeutic agent for coronavirus infection according to any one of items 5 to 7, the ACE2 and TMPRSS2 expression inhibitor according to item 8, or the IL6 AMP inhibitor according to item 9, wherein the inhibitor or the agent is an inhalant.

### Advantageous Effects of Invention

The present invention can provide a novel STAT3 phosphorylation and IL6 AMP inhibitor. In addition, the present invention provides a novel prophylactic or therapeutic agent for a disease that can be treated by inhibiting STAT3 phosphorylation. For example, the present invention provides a novel prophylactic or therapeutic agent for autoimmune disease, the agent containing a substance that has not been known for its therapeutic effect on autoimmune disease as an active ingredient. Furthermore, the present invention can provide a novel prophylactic or therapeutic agent for coronavirus infection using STC1, a substance that has not been known for its therapeutic effect on coronavirus infection, as an active ingredient. Specifically, according to the present invention, the agent can inhibit infection with a coronavirus through inhibition of the expression of ACE2 and TMPRSS2 and thus is useful for prevention and treatment of coronavirus infection. In addition, the present inventors already found that STC1 significantly inhibits lung fibrogenesis (Patent Literature 1). Thus, according to the present invention, the agent can be expected not only to prevent or treat coronavirus infection but also to prevent lung fibrogenesis also when coronavirus infection is aggravated, and to have a therapeutic effect and progression inhibitory effect on lung fibrogenesis also when it occurs, and thus is further useful. Furthermore, such effects of the present invention is unexpected because an agent capable of inhibiting pulmonary fibrosis does not necessarily prevent or treat coronavirus infection.

### BRIEF DESCRIPTION OF DRAWINGS

FIG. 1 is a diagram illustrating an overview of a bleomycin (BLM) intratracheal instillation lung injury model of a mouse.
FIG. 2 is photos and graphs showing results of Western blotting and quantitative PCR in Example 1.
FIG. 3 is a diagram illustrating an overview about effects of STC1 on the mitochondrial TCA cycle and its peripheral metabolism.
FIG. 4 is photos and graphs showing results of Western blotting in Example 2.
FIG. 5 is a graph showing results of semi-quantitative methylation-specific PCR in Example 2.
FIG. 6 is photos showing measurement results of the degree of acetylation of SMAD7 protein in Example 2.
FIG. 7 is photos showing results of hematoxylin-eosin staining in Example 3.

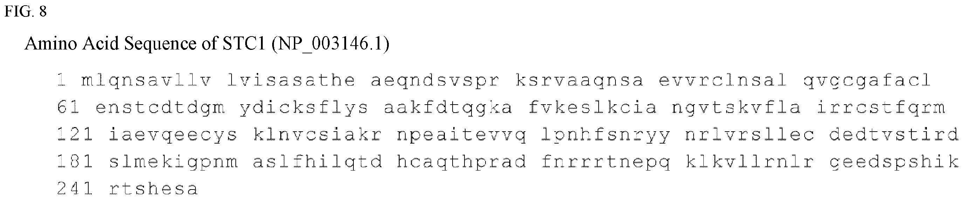

FIG. 8 is an amino acid sequence of human STC1 registered under registration number NP_003146.1 shown in SEQ ID NO: 1.
FIG. 9 is photos showing results of Western blotting in Example 4-1).
FIG. 10 is photos showing results of Western blotting in Example 4-2).
FIG. 11 is photos and graphs showing results of Western blotting (A), semi-quantitative PCR (B), and semi-quantitative methylation-specific PCR in Example 4-3).
FIG. 12 is photos showing results of Western blotting in Example 4-4).
FIG. 13 is photos showing results of immunoprecipitations in Example 4-5).
FIG. 14 is photos showing results of Western blotting in Example 4-6).
FIG. 15 is photos showing results of Western blotting in Example 4-7).
FIG. 16 is photos showing results of an animal experiment in Example 4-8).
FIG. 17 is a diagram illustrating an outline of findings elucidated in the examples.

### DESCRIPTION OF EMBODIMENTS

### STAT3 Phosphorylation Inhibitor

The present invention provides a STAT3 phosphorylation inhibitor containing stanniocalcin 1 as an active ingredient. Signal transducer and activator of transcription 3 (STAT3) is a protein belonging to the STAT protein family and is known to be phosphorylated and activated in the IL-6/JAK/STAT transduction pathway or the like. Typically, examples of STAT3 include those whose amino acid sequence is registered in NCBI accession No. NP_001356441.1.

STC1, an active ingredient of the present invention, is a known component described in Patent Literature 1 and the like and is a dimeric glycoprotein hormone. In the present invention, STC1 may be a naturally occurring STC1 molecule obtained from an animal cell or tissue, or the like, recombinant STC1 having an identical amino acid sequence to that of naturally occurring STC1, recombinant STC1 produced based on an STC1 gene modified by genetic engineering technology, or synthesized STC1. In addition, in the present invention, STC1 may be a functional equivalent or the like, such as an active fragment of STC1. Thus, in the present invention, the term "stanniocalcin 1" (STC1) is used in a meaning including these various STC1s unless otherwise stated.

In addition, STC1 used in the present invention is preferably one having an amino acid sequence derived from a human or one modified based on an amino acid sequence derived from a human without impairing the function (e.g., one in which one or several (e.g., two, two or three, two to four, or two to five) amino acids are deleted, added, and/or replaced). The amino acid sequence and base sequence of human STC1 are known and registered under registration numbers NP#003146.1 and NM#003155.2. For example, the amino acid sequence of human STC1 registered under registration number NP_003146.1 is shown in SEQ ID NO: 1. In addition, amino acid sequences and base sequences are also known for many STC1s derived from an animal other than a human. These are all known to have high homology to human STC1 (Table 1).

**TABLE 1**

| NCBI Ref Seq | Species | Species (common name) | Query cover | Identity |
|---|---|---|---|---|
| NP_003146.1 | Homo sapiens | Human | 100 | 100 |
| XP_002818956.1 | Pongo abelii | Sumatran Orangutan | 100 | 100 |
| XP_003823619.1 | Pan paniscus | Bonobo | 100 | 100 |
| XP_004046836.1 | Gorilla | Gorilla | 100 | 100 |
| XP_528091.1 | Pan troglodytes | Chimpanzee | 100 | 100 |
| XP_543238.3 | Canis familiaris | Dog | 100 | 98.4 |
| NP_788842.2 | Bos taurus | Cattle | 100 | 95.1 |
| NP_112385.1 | Rattus norvegicus | Rat | 100 | 96 |
| NP_033311.3 | Mus musculus | Mouse | 100 | 96.4 |
| XP_001506746.1 | Ornithorhynchus anatinus | Platypus | 100 | 88.3 |
| XP_004947617.1 | Gallus | Chicken | 95 | 88.9 |
| AAV58802.1 | Platichthys flesus | Plaice | 96 | 72.1 |
| XP_019968668.1 | Paralichthys olivaceus | Flounder | 96 | 70.45 |
| XP_020318608.1 | Oncorhynchus kisutch | Coho salmon | 98 | 59.1 |
| XP_020318608.1 | Oncorhynchus mykiss | Rainbou Traut | 98 | 59.1 |
| NP_001088352.1 | Xenopus laevis | African clawed frog | 73 | 62.3 |
| XP_019412118.1 | Crocodylus porosus | Saltwater crocodile | 89 | 54.7 |
| XP_019338001.1 | Alligator mississippiensis | American alligator | 89 | 54.7 |

Thus, in one embodiment of the present invention, examples of STC1 that can be used include those having a homology of 80% or higher, preferably 90% or higher, and more preferably 94% or higher to the amino acid sequence of human STC1. Typically, naturally occurring or recombinant STC1 (rSTC1) having an amino acid sequence 100% identical to a human STC1 sequence (e.g., a human STC1 sequence derived from 293 cells) is even more preferably used. In the present invention, one of these STC1s can be used individually or two or more STC1s can be used in combination.

STC1 and its salt, active ingredients of the present invention, may present in the form of hydrate or solvate, and thus these hydrate and solvate are also included in a compound of the active ingredient of the present invention.

The solvent forming the solvate is exemplified by alcohols, such as ethanol and propanol; organic acids, such as acetic acid; esters, such as ethyl acetate; ethers, such as tetrahydrofuran and diethyl ether; ketones, such as acetone; and DMSO. One of these solvents can be used individually or two or more solvents can be used in combination.

In the present invention, STC1 itself, the active ingredient of the present invention, may be used as the STAT3 phosphorylation inhibitor or may be used in a pharmaceutical composition in which STC1 is combined with a pharmaceutically acceptable carrier of various types (e.g., such as an isotonic agent, a chelating agent, a stabilizer, a pH adjuster, an antiseptic, an antioxidant, a solubilizer, or a thickening agent).

Examples of the isotonic agent include saccharides, such as glucose, trehalose, lactose, fructose, mannitol, xylitol, and sorbitol; polyhydric alcohols, such as glycerin, poly(ethylene glycol), and propylene glycol; and inorganic salts, such as sodium chloride, potassium chloride, and calcium chloride. One of these isotonic agents can be used individually or two or more isotonic agents can be used in combination.

Examples of the chelating agent include edetate salts, such as disodium edetate, calcium disodium edetate, trisodium edetate, tetrasodium edetate, and calcium edetate; ethylenediaminetetraacetate salts; nitrilotriacetic acid or its salt; sodium hexametaphosphate; and citric acid. One of these chelating agents can be used individually or two or more chelating agents can be used in combination.

Examples of the stabilizer include sodium hydrogen sulfite.

Examples of the pH adjuster include acids, such as hydrochloric acid, carbonic acid, acetic acid, and citric acid; and further include bases, such as alkali metal hydroxides including sodium hydroxide and potassium hydroxide; alkali metal carbonates including sodium carbonate; or hydrogen carbonates; alkali metal acetates including sodium acetate; alkali metal citrates including sodium citrate; and trometamol. One of these pH adjusters can be used individually or two or more pH adjusters can be used in combination.

Examples of the antiseptic include sorbic acid, potassium sortable; p-hydroxybenzoate, such as methyl p-hydroxybenzoate, ethyl p-hydroxybenzoate, propyl p-hydroxybenzoate, and butyl p-hydroxybenzoate; chlorhexidine gluconate; quaternary ammonium salts, such as benzalkonium chloride, benzethonium chloride, and cetyl pyridinium chloride; alkylpolyaminoethylglycine; chlorobutanol; polyquad; poly(hexamethylene biguanide); and chlorhexidine. One of these antiseptics can be used individually or two or more antiseptics can be used in combination.

Examples of the antioxidant include sodium hydrogen sulfite, exsiccated sodium sulfite, sodium pyrosulfite, and concentrated mixed tocopherol. One of these antioxidants can be used individually or two or more antioxidants can be used in combination.

Examples of the solubilizer include sodium benzoate, glycerin, D-sorbitol, glucose, propylene glycol, hydroxypropyl methylcellulose, polyvinylpyrrolidone, macrogol, and D-mannitol. One of these solubilizers can be used individually or two or more solubilizers can be used in combination.

Examples of the thickening agent include poly(ethylene glycol), methyl cellulose, ethyl cellulose, carmellose sodium, xanthan gum, sodium chondroitin sulfate, hydroxyethyl cellulose, hydroxypropyl cellulose, hydroxypropyl methylcellulose, polyvinylpyrrolidone, and poly(vinyl alcohol). One of these thickening agents can be used individually or two or more thickening agents can be used in combination.

In an embodiment of the pharmaceutical composition, the content of STC1 in the composition is not particularly limited and can be appropriately set from conditions, such as 90 mass% or higher, 70 mass% or higher, 50 mass% or higher, 30 mass% or higher, 10 mass% or higher, 5 mass% or higher, and 1 mass% or higher.

The preparation form is not particularly limited, and examples include various preparation forms, such as orally administered agents including tablets, pills, capsules, powders, granules, and syrups; and parenterals including injections (such as intravenous injections, intramuscular injections, and local injections), gargles, infusions, external preparations (ointments, creams, patches, and inhalants), and suppositories. Preferred examples among the above preparation forms include external preparations, in particular, inhalants (intratracheally administered agents).

In the present invention, the dose of STC1 varies with administration route, age, body weight, and symptoms of patients, and the like and cannot be unconditionally specified, but the daily dose to an adult human is commonly about 5000 mg or lower and preferably about 1000 mg or lower. The lower limit of the dose of STC1 is not particularly limited either. For example, the dose of STC1 can be appropriately set commonly in the range of 0.1 mg or higher and preferably 0.5 mg or higher in terms of daily dose to an adult human. One preparation is to contain this amount when STC1 is administered once daily, and one preparation is to contain a third of this amount when STC1 is administered three times daily.

The STAT3 phosphorylation inhibitor of the present invention is administered to patients, such as those of mammals. Examples of the mammals include humans, monkeys, mice, rats, rabbits, cats, dogs, pigs, cattle, horses, and sheep.

### Prophylactic or Therapeutic Agent for Autoimmune Disease

The present invention provides a prophylactic or therapeutic agent for autoimmune disease, the agent containing stanniocalcin 1 as an active ingredient. A cytokine, such as IL6, when binding to a receptor, induces the phosphorylation of JAK and STAT, and this induces transcription of an acute phase protein (Non-Patent Literature 5). SOCS inhibits the phosphorylation of JAK and thus indirectly inhibits the phosphorylation of STAT as well. The IL6/JAK/STAT pathway is considered an important pathway involved in the onset of autoimmune disease, such as rheumatoid arthritis, and has been targeted for drug discovery. Here, as described above, the present inventors have found this time that STC1 inhibits the phosphorylation of STAT3 located downstream of the IL-6/JAK/STAT transduction pathway. The present invention is based on these new findings. In addition to STC1, the active ingredient of the present invention, its hydrate and solvate can also be used as described above, and others are as described above.

With respect to the target disease in the present invention, examples of autoimmune disease include rheumatoid arthritis, Castleman's disease, Crohn's disease, Wegener granulomatosis, sarcoidosis, atopic dermatitis, chronic pyoderma, psoriasis, and hyper-IgE syndrome associated with pneumonia.

In the present invention, STC1 itself, the active ingredient of the present invention, may be used as the prophylactic or therapeutic agent for autoimmune disease or may be used in a pharmaceutical composition in which STC1 is combined with a pharmaceutically acceptable carrier of various types. A carrier of those described for the STAT3 phosphorylation inhibitor can be similarly used in the amount described above.

In addition, the pharmaceutical composition may further contain a compound reported to have a prophylactic or therapeutic effect on autoimmune disease in addition to STC1. Examples of the compound known to have a prophylactic or therapeutic effect on autoimmune disease include corticosteroids (such as prednisolone), JAK inhibitors (such as tofacitinib), calcineurin inhibitors (such as cyclosporin/tacrolimus), DNA synthesis inhibitors (such as cyclophosphamide), mTOR inhibitors (such as sirolimus), IMDH inhibitors (such as mycophenolate mofetil), and antibody preparations (such as tocilizumab and rituximab). One of these compounds can be used individually or two or more compounds can be used in combination. The content of STC1 in the composition, preparation form, dose, patient eligible for administration, and the like in an embodiment of the pharmaceutical composition are similar to those described above for the STAT3 phosphorylation inhibitor.

### Prophylactic or Therapeutic Agent for Coronavirus Infection

The present invention provides a prophylactic or therapeutic agent for coronavirus infection, the agent containing STC1. In addition to STC1, the active ingredient of the present invention, its hydrate and solvate can also be used as described above, and others are as described above.

With respect to the target disease in the present invention, the coronavirus is intended to be a coronavirus belonging to the Coronaviridae. Examples of the coronavirus include the genus Alphacoronavirus and the genus Betacoronavirus and preferably include the genus Betacoronavirus. Examples of the genus Alphacoronavirus include the subgenus Duvinacovirus (e.g., such as HCoV-229E) and the subgenus Setracovirus (e.g., such as HCoV-NL63). Examples of the genus Betacoronavirus include the subgenus Embecovirus (e.g., such as HCoV-OC43 and HCoV-HKU1), the subgenus Sarbecovirus, the subgroup Merbecovirus (e.g., such as Middle East respiratory syndrome coronavirus (MERS-CoV)) and preferably include the subgenus Sarbecovirus. Examples of the subgenus Sarbecovirus include severe acute respiratory syndrome coronavirus (SARSr-CoV). Examples of the SARSr-CoV include severe acute respiratory syndrome coronavirus (SARS-CoV) and severe acute respiratory syndrome coronavirus 2 (SARS-CoV2).

The coronavirus, such as SARS-CoV2, has a spike protein (S protein) on its envelope, and the S protein binds to the receptor (ACE2) of the cell membrane, then undergoes proteolysis by transmembrane protease serine 2 (TMPRSS2), a proteolytic enzyme, and the S protein is activated. This is known to be important for the fusion of the viral outer membrane and the cell membrane, and ACE2 and TMPRSS2 are considered mandatory in the airway cells for the infection with SARS-CoV-2 (Non-Patent Literatures 1 and 2). The present inventors have found that while lung injury otherwise increases the expression of ACE2 and TMPRSS2, using STC1, the active ingredient of the prophylactic or therapeutic agent of the present invention, inhibits the increase in the expression of ACE2 and TMPRSS2 in the lung injury. Thus, the prophylactic or therapeutic agent for coronavirus infection is particularly effective against a coronavirus having an S protein, typically a coronavirus having an S protein capable of binding to ACE2.

In the present invention, STC1 itself, the active ingredient of the present invention, may be used as the prophylactic or therapeutic agent for coronavirus infection or may be used in a pharmaceutical composition in which STC1 is combined with a pharmaceutically acceptable carrier of various types. A carrier of those described for the STAT3 phosphorylation inhibitor can be similarly used.

In addition, the pharmaceutical composition may further contain a compound reported to have a prophylactic or therapeutic effect on coronavirus infection in addition to STC1. Examples of the compound known to have a prophylactic or therapeutic effect on coronavirus infection include favipiravir, remdesivir, ivermectin, ciclesonide, tocilizumab, camostat, and nafamostat. One of these compounds can be used individually or two or more compounds can be used in combination. The content of STC1 in the composition, preparation form, dose, patient eligible for administration, and the like in an embodiment of the pharmaceutical composition are similar to those described above for the STAT3 phosphorylation inhibitor.

STC1, the active ingredient of the prophylactic or therapeutic agent for coronavirus infection, prevents, treats, and/or relieves coronavirus infection by inhibiting at least the expression of ACE2 and TMPRSS2. Thus, the present invention also provides an ACE2 and TMPRSS2 expression inhibitor. In addition, the present inventors found that STC1 significantly inhibits lung fibrogenesis, and a pharmaceutical composition for prevention, treatment, and/or progression inhibition of pulmonary fibrosis, the composition containing STC1 as an active ingredient, has been patented (Patent Literature 1). However, although lung fibrogenesis may occur when coronavirus infection becomes severe, it is known that lung fibrogenesis can be caused by a wide variety of factors, and coronavirus infection is merely one of them, and a compound capable of treating lung fibrogenesis appears not necessarily to inhibit the infection itself with a coronavirus. In addition, the relationship between the expression of ACE2 and TMPRSS2 and STC1 has never been reported to date. Thus, the inhibitory effect of STC1 on the expression of ACE2 and TMPRSS2 and the prophylactic and treatment effects of STC1 on coronavirus infection in the present invention cannot be expected from the related art. In addition, TMPRSS2 has been reported to play an important role also in influenza (type A and type B) and infections with other respiratory-related viruses or the like (Non-Patent Literature 6). Thus, the ACE2 and TMPRSS2 expression inhibitor of the present invention can also be used as a prophylactic or therapeutic agent for a respiratory-related virus (e.g., coronavirus or influenza virus) or the like. The active ingredient, preparation form, dose, and the like of the ACE2 and TMPRSS2 expression inhibitor and the prophylactic or therapeutic agent for a respiratory-related virus are similar to those of the prophylactic or therapeutic agent for coronavirus infection.

### IL-6 AMP Inhibitor

When SARS-CoV2 binds to ACE2, angiotensin 2, which otherwise binds to ACE2 and is degraded, induces TNFα, IL6, and the like through a receptor called AT1R. As a result, STAT3 is activated, and synthesis of IL6 is further increased. This is called IL6 AMP as IL6 itself amplifies IL6 and is considered to be involved in causing fulminant COVID19. In addition, the pathway in which the virus is recognized by innate immunity and NfkB is activated is also considered to work additively on IL6 AMP. As described above, STC1, the active ingredient of the present invention, inhibits IL-6 AMP by inhibiting the IL-6/JAK/STAT transduction pathway. Thus, the present invention also provides an IL-6 AMP inhibitor containing STC1. The active ingredient, preparation form, dose, and the like of the IL-6 AMP inhibitor are similar to those described above.

Hereinafter, the present invention will be described more specifically with reference to examples, but the present invention is not limited to these.

### EXAMPLES

In the present examples, human recombinant (r) STC1 was manufactured by BioVender. Specifically, a human-derived STC1 gene in which a TAG structure was added was introduced into 293 cells, and rSTC1 was separated using the TAG sequence. In the following examples, the human recombinant (r) STC1 may be represented simply as STC1.

### Example 1

### Intratracheal Instillation of STC1 Inhibits Expression of ACE2 and TMPRSS2 in Bleomycin Lung Injury Model

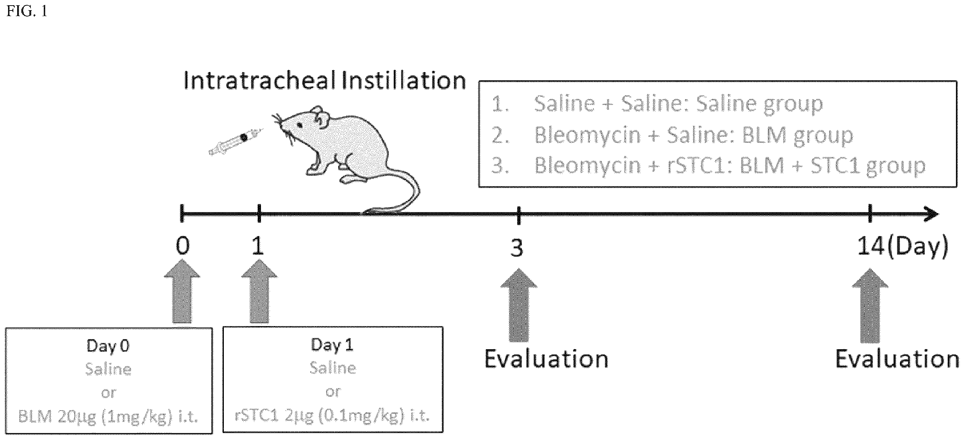

In the present example, a bleomycin (BLM) intratracheal instillation lung injury model of a mouse was used (FIG. 1). Specifically, on Day 0, six C57BL6/J mice (female, 8-week old) each per group were anesthetized by intraperitoneally giving anesthetics (300 µg of ketamine hydrochloride and 160 ng of xylazine diluted in 100 µL of saline). The cervix was then incised to expose the trachea, and a catheter of a 23G Surflo needle was inserted to the trachea. Furthermore, the following agents were each instilled from the Surflo catheter into the mouse trachea of each group.
(i) Saline group (control): 50 µL of saline
(ii) BLM group: 50 µL of bleomycin-containing saline (bleomycin concentration 0.4 mg/mL)
(iii) BLM + STC1 group: 50 µL of bleomycin-containing saline (bleomycin concentration 0.4 mg/mL)

On Day 1, the mice were anesthetized in the same manner as described above, and the following agents were each instilled from the Surflo catheter into the mouse trachea in each group.
(i) Saline group (control): 50 µL of saline
(ii) BLM group: 50 µL of saline
(iii) BLM + STC1 group: 50 µL of STC1-containing saline (STC1 concentration 0.4 mg/mL)

On Day 3, three mice per each group were anesthetized in the same manner as described above and euthanized by cutting the carotid arteries, and then the lungs were excised. Furthermore, on Day 14, three mice per each group were anesthetized in the same manner as described above and euthanized by cutting the carotid arteries, and then the lungs were excised.

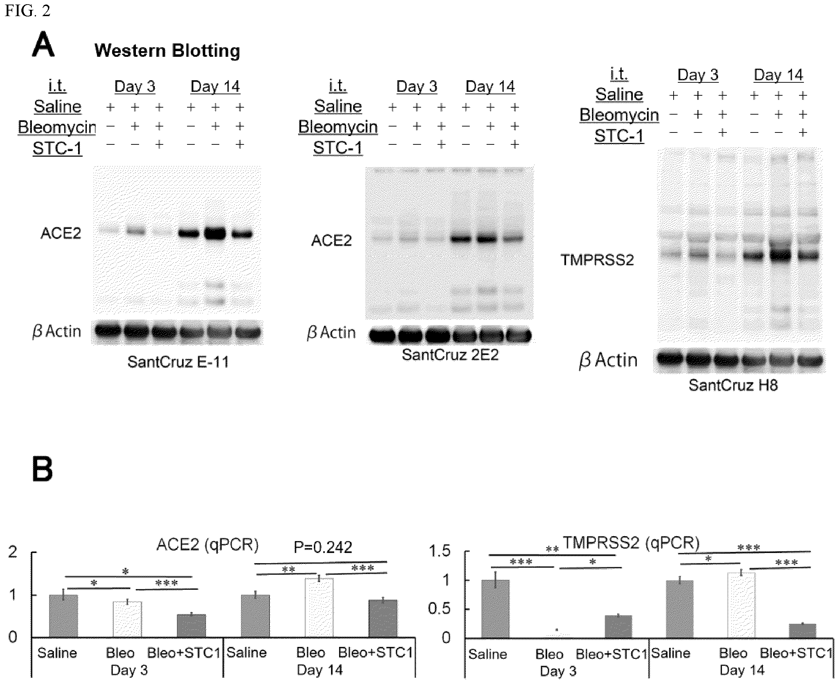

The excised lungs were subjected to Western blotting. For antibodies, anti-ACE2 antibodies (E-11 and 2E2) and an anti-TMPRSS2 antibody (H8) from Santa Cruz were used. The results of Western blotting of each group are shown in FIG. 2A. As shown in FIG. 2A, STC1 strongly inhibited the increases in ACE2 and TMPRSS2 induced by bleomycin lung injury.

In addition, quantitative PCR was performed using an ABI 7500 Real Time PCR system from Applied Biosystem. The results are shown in FIG. 2B. As shown in FIG. 2B, lung injury increased the expression of ACE2 and TMPRSS2 (comparison of Saline group and Bleo group). In addition, as is clear from the comparison of Bleo group and Bleo + STC1 group in FIG. 2B, the intratracheal instillation of STC1 inhibited the increase in the expression of ACE2 and TMPRSS2 in lung injury.

As described above, the presence of ACE2 and TMPRSS2 in airway cells is mandatory for infection with a coronavirus, and thus STC1 appears to be effective in prevention and treatment of infection with a coronavirus from the results described above. In addition, lung injury increased the expression of ACE2 and TMPRSS2 as described above, and thus this presumably causes a vicious cycle in which lung injury renews the expression of ACE2 and TMPRSS2, allowing coronaviruses to enter cells easily through ACE2 and TMPRSS2 and further aggravating the coronavirus infection. According to the present invention, inhibition of the expression of ACE2 and TMPRSS2 can inhibit the progression of the vicious cycle, and thus this is useful.

### Example 2

### Investigation of Metabolic Alteration Effect of STC1 by Metabolomic Analysis

### Effects of STC1 on SMAD 2, 3, and 7

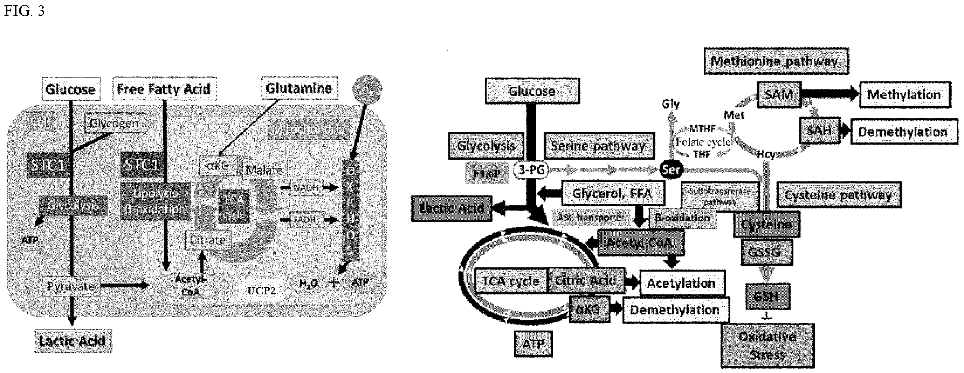

Effects of STC1 on the mitochondrial TCA cycle and its peripheral metabolism are illustrated in FIG. 3.

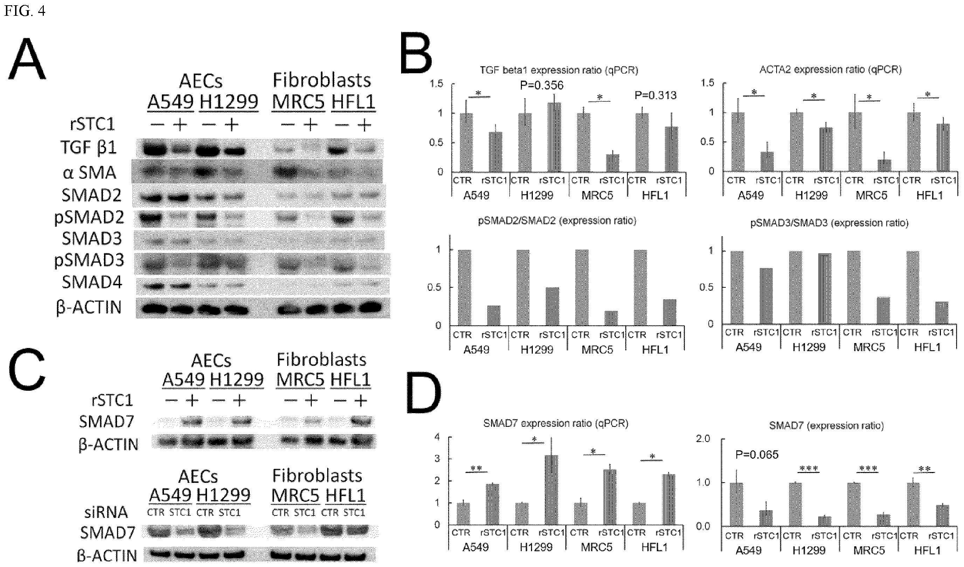

Effects of STC1 on the TGFβ/SMAD signal transduction system were analyzed by Western blotting and quantitative PCR. Specifically, human alveolar epithelial cell lines A549, H1299, human fibroblast cell lines MRC5, and HFL1 were cultured in a recombinant STC1 (50 ng/mL)-supplemented or non-supplemented cell culture for 24 hours. For antibodies, anti-SMAD7 antibody (B8) from Santa Cruz and a Phospho-Smad Andibody Sampler Kit from Cell Signaling Technology were used. For the quantitative PCR, an ABI 7500 Real Time PCR system from Applied Biosystem was used. The results are shown in FIGS. 4A to D.

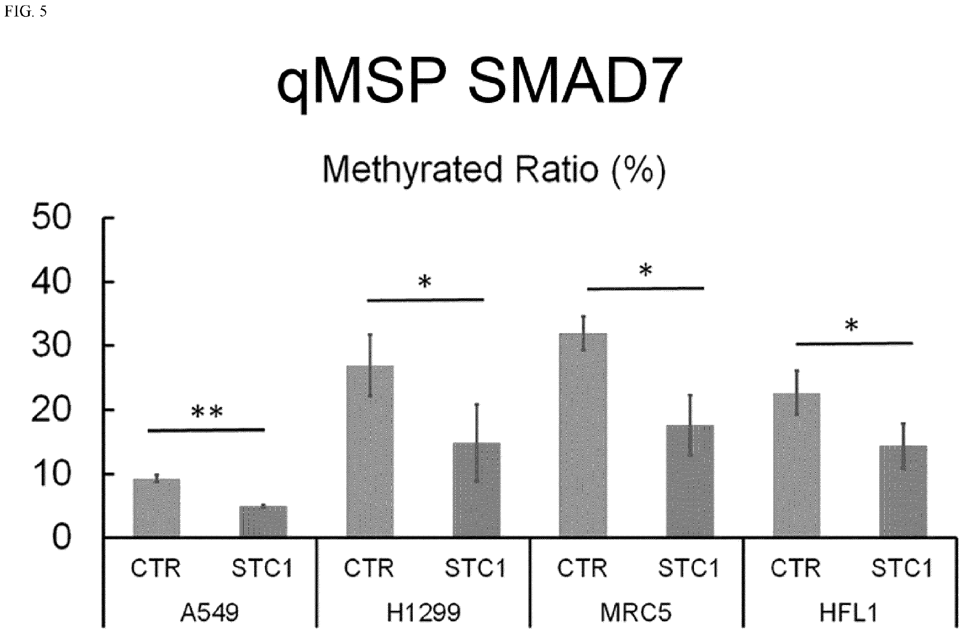

As shown in FIG. 4, STC1 inhibited the phosphorylation of SMAD2/3 required for lung injury and lung fibrogenesis. This effect was due to induction of SMAD7, an inhibitory SMAD, by STC1. Excessive and persistent secretion of TGF beta induces chronic anti-oxidative stress and inflammatory cytokine through SMAD2/3. These are involved in the cytokine storm responsible for the aggravation of coronavirus infection, such as COVID19 (such as lung injury). Thus, the above results suggest that inhibition of SMAD 2/3 phosphorylation by STC1 inhibits the persistent secretion of inflammatory cytokines, and this can avoid lung injury in coronavirus infection and severe pneumonia. Effects of STC1 on methylation of the promoter region of the SMAD7 gene are shown in FIG. 5. The effects of STC1 on methylation of the promoter region of the SMAD7 gene were analyzed by quantitative methylation-specific PCR (qMSP). Specifically, genomic DNA extracted from each cell was bisulfite-treated (Fujifilm-Wako, EpiSight^{™} Bisulfite Conversion Kit Ver. 2), and the qMSP was performed using a polymerase (Takara Episcope MSP kit) for methylation-specific PCR and an ABI 7500 Real Time PCR system. As shown in FIG. 5, STC1 also induced demethylation of the SMAD7.

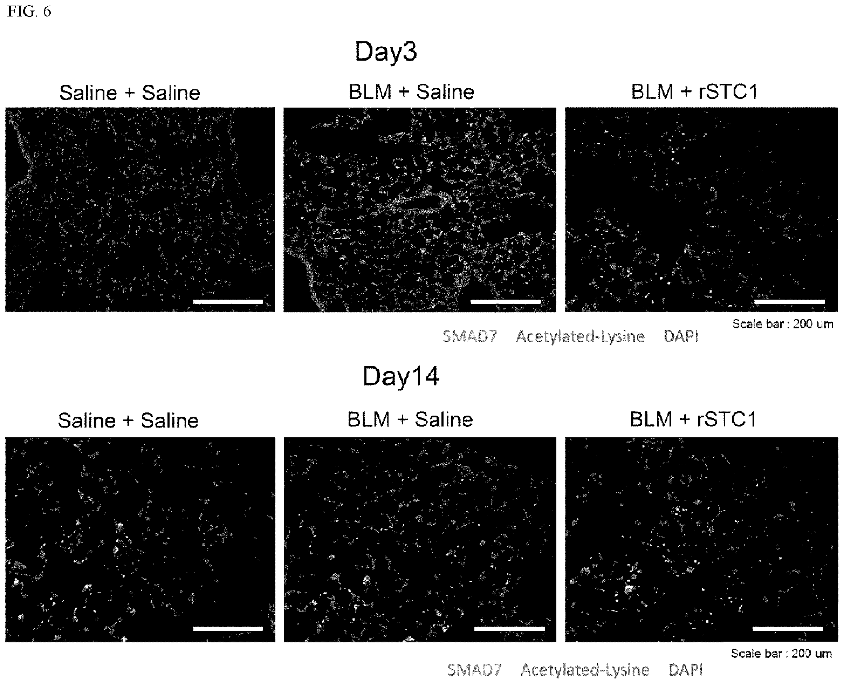

Effects of STC1 on acetylation of the SMAD7 protein are shown in FIG. 6. The effects of STC1 on SMAD7 of the lung of a mouse were analyzed by immunofluorescence staining of excised lungs of mice. Specifically, the effects were investigated by slicing the lung used in Example 1 and fixing the sliced lung on a preparation plate, combining an Alexa-488 antibody and an Alexa 594 antibody from Molecular Device with an SMAD7 antibody (B8 from Santa Cruz), an anti-acetylated lysine antibody (Ac-K-103 from Cell Signaling Technology, Inc.), and a nuclear stain (DAPI from DACO), and using a phase-contrast microscope.

The results are shown in FIG. 6. As shown in FIG. 6, STC1 also induced acetylation of SMAD7 lysine groups. The acetylation enables stable expression of SMAD7. That is, STC1 turned out to increase the expression of SMAD7 through epigenetic regulation of SMAD7.

### Example 3

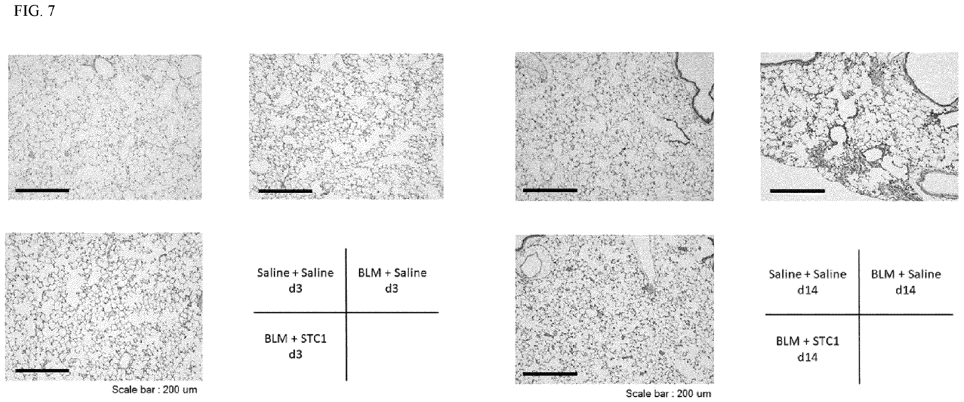

In addition, the lung specimens excised from mice in each group obtained in Example 1 were used for hematoxylin-eosin staining. The results are shown in FIG. 7. As shown in FIG. 7, STC1 inhibited the inflammation and fibrogenesis of the lung due to bleomycin.

### Example 4

### Evaluation of Effects of STC1 on STAT3 Phosphorylation, IL-6 AMP, and Others

### <Methods>

### • Cell Experiment

Human-derived monocytic THP1 cells were cultured in RPMI medium (Sigma) containing 10% fetal bovine serum (Nichirei) and 1% penicillin-streptomycin (Sigma) for 24 hours and then differentiated into macrophages by supplementing 10 uM of phorpol 12-myristate 13 acetate (PMA). Twenty-four (24) hours after the addition of PMA, the following three groups were prepared: a group not supplemented with human recombinant IL6, a group supplemented with 10 ng/mL of IL6, and a group supplemented with 50 ng/mL of IL6. Furthermore, they were not supplemented or supplemented with 50 ng/mL of STC1, that is, 6 groups of 3 × 2 were prepared. The cells were cultured under those conditions for 24 hours and then subj ected to Western blot, immunoprecipitation, PCR, methylation-specific PCR, and inhibition of specific gene transcription using siRNA.

### • Animal Experiment

In the present example, a bleomycin (BLM) intratracheal instillation lung injury model of a mouse was used.

Specifically, according to the method described in Example 1, bleomycin (BLM) intratracheal instillation lung injury model mice were prepared, and the lungs were excised from three mice per each group on Day 3 and Day 14.

### • Method of Western Blotting

After electrophoresis with the THP1 cell total extract or the excised mouse lung extract with RIPA buffer and SDS-PAGE gel, cells or tissue-derived proteins were transferred to a PVDF membrane and incubated with a primary antibody and an HRP-conjugated secondary antibody. Color was then developed with a chromogenic substrate, and protein expression was measured with a chemiluminometer.

The primary antibodies used in Western blotting are as follows:
Anti-STAT3 antibody (Cell Signalling Technology, Inc. (CST), 12640), anti-pSTAT3 antibody (CST, 9145), anti-SOCS1 antibody (CST, 3950), anti-IL6 antibody (CST, 12153), anti-JAK1 antibody (CST, 3344), anti-pJAK1 antibody (CST, 74129) antibody, anti-Ubiquitin antibody (CST, 58395), anti-ACE2 antibody (Santa Cruz (SC), 390851), anti-TMPRSS2 antibody (SC, 515727), and anti-β ACTIN antibody (R&D, MAB8929). For the secondary antibody, an HRP-conjugated antibody compatible with each animal species was used.

### • Method of Immunoprecipitation

Protein A/G plus agarose (SC, 2003) and an anti-JAK1 antibody (SC, 1677) were bound, then THP1 cell total extract or excised mouse lung extract with RIPA buffer was added, and JAK1 in these extracts and proteins bound to JAK1 were collected. The JAK1 protein and protein bound to JAK1 were separated by boiling with a reducing solution.

### • Semi-Quantitative PCR

mRNA was separated from THP1 cells and mouse lung tissues using an RNeasy Mini Kit (QIAGEN), and cDNA was then prepared by reverse transcription from the mRNA with a High Capacity cDNA Kit (Applied Biosystems). The primer was designed using a primer blast (NCBI), and semi-quantitative PCR was performed using SYBR Green PCR Master Mix and ABI 7500 Real-time PCR System (Applied Biosystems).

### • Methylation-Specific PCR

Methylated and demethylated primers of the promoter region of the SOCS1 gene were designed using Methyl Primer Express software (Applied Biosystems). Genomic DNA extracted from THP1 cells using a DNeasy Blood & Tissue Kit (QIAGEN) was bisulfite-treated (Fujifilm-Wako, EpiSight^{™} Bisulfite Conversion Kit Ver. 2), and the methylation-specific PCR was performed using a polymerase (Takara Episcope MSP kit) for methylation-specific PCR and an ABI 7500 Real Time PCR system.

### • siRNA

For the SOCS1-specific siRNA and control siRNA, predesigned siRNAs (Ambion) were used. For the transfection reagent, Lipofectamine RNAiMAX (Invitrogen) was used.

### <Results>

### (THP1 Cell Data)

### 1) STC1 Strongly Inhibits IL6-Induced STAT3 Phosphorylation in THP1 Cells

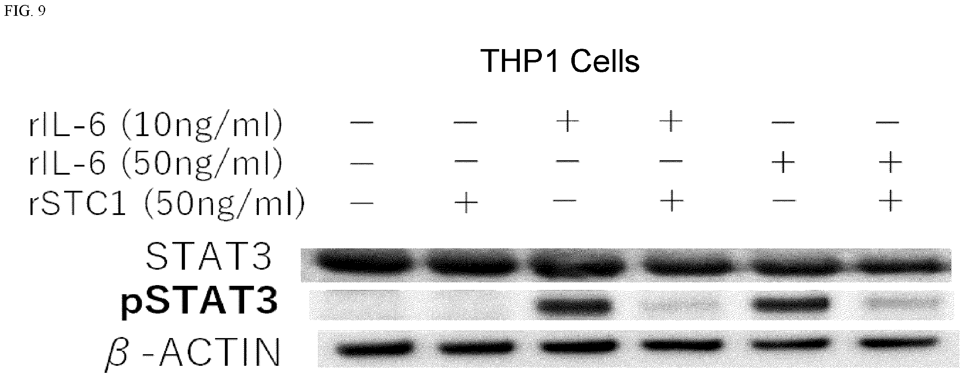

The presence or absence of STAT3 and phosphorylated STAT3 in the cell total extracts of the THP1 cells supplemented with IL6 (10 ng/mL), IL6 (50 ng/mL), and/or STC1 (50 ng/mL) as well as the THP1 cells (control) not supplemented with these according to the above method was measured by Western blotting. The results are shown in FIG. 9. As shown in FIG. 9, STC1 inhibited STAT3 phosphorylation induced by IL6.

That is, STC1 inhibits IL6-induced STAT3 phosphorylation and thus is useful in treatment of cytokine syndromes and various autoimmune diseases.

### 2) STC1 Inhibits IL6-Induced IL6 Production Amplification (IL6 AMP) in THP1 Cells

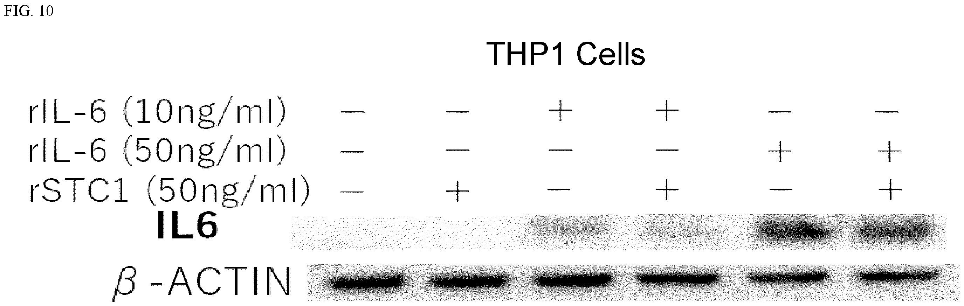

The presence or absence of IL6 in the cell total extracts of the THP1 cells supplemented with IL6 (10 ng/mL), IL6 (50 ng/mL), and/or STC1 (50 ng/mL) as well as the THP1 cells (control) not supplemented with these according to the above method was measured by Western blotting. The results are shown in FIG. 10. As shown in FIG. 10, STC1 inhibited IL6 production amplification (IL6 AMP) induced by IL6.

That is, STC1 inhibits IL6-induced IL6 amplification (IL6 AMP) and thus is useful in treatment of cytokine syndromes and various autoimmune diseases.

### 3) STC1 Induces SOCS1 in THP1 Cells

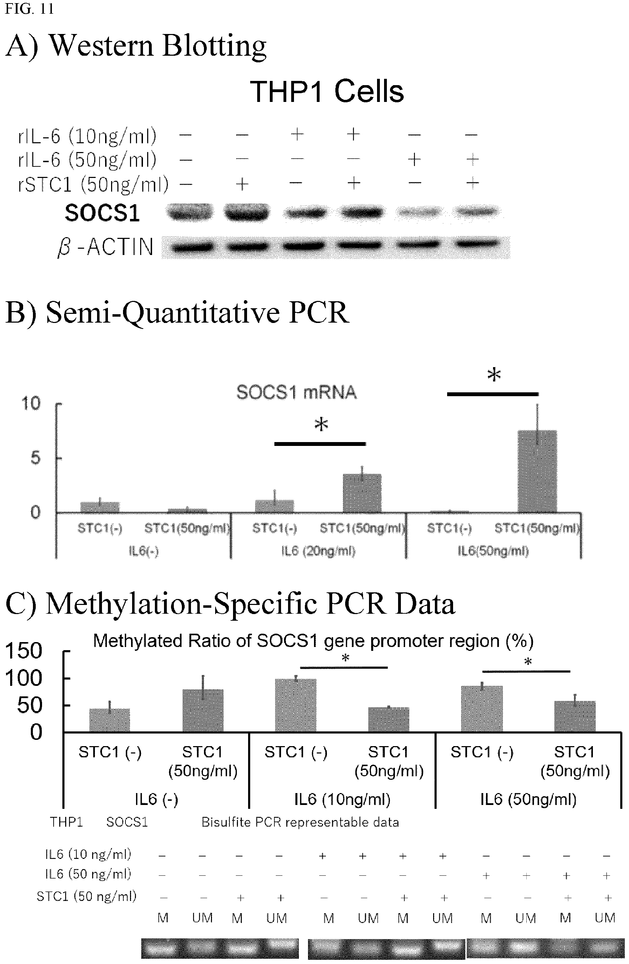

The THP1 cells supplemented with IL6 (10 ng/mL), IL6 (50 ng/mL), and/or STC1 (50 ng/mL) as well as the THP1 cells (control) not supplemented with these according to the above method were subjected to the following, and the results are shown in FIGS. 11A to C: Western blotting of SOCS1 in the cell total extracts of the above THP1 cells (FIG. 11A); the semi-quantitative PCR method of SOCS1 mRNA using the cell total extract mRNAs of the above THP1 cells (FIG. 11B); and measurement of the degree of methylation of the SOCS1 gene promoter region using the cell total extraction DNAs of the above THP1 cells by the methylation-specific PCR method (FIG. 11C).

As shown in FIG. 11A, STC1 increased the production of SOCS1 protein. In addition, as shown in FIG. 11B, STC1 increased the production of mRNA of SOCS1. As shown in FIG. 11C, STC1 inhibited the methylation of the SOCS1 gene promoter region. The results in FIGS. 11A to C all mean the production increase of SOCS1 protein by STC1.

That is, STC1 induces SOCS1 having an effect of inhibiting JAK and thus is useful in treatment of cytokine syndromes and various autoimmune diseases.

### 4) STC1 Inhibits Expression and Phosphorylation of JAK1 in THP1 Cells

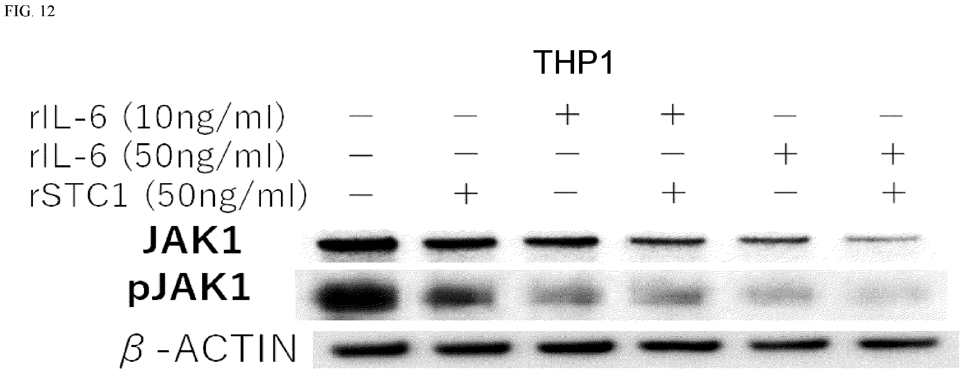

The presence or absence of JAK1 and JAK1 phosphorylation in the cell total extracts of the THP1 cells supplemented with IL6 (10 ng/mL), IL6 (50 ng/mL), and/or STC1 (50 ng/mL) as well as the THP1 cells (control) not supplemented with these according to the above method was measured by Western blotting. The results are shown in FIG. 12. As shown in FIG. 12, STC1 inhibited the expression and phosphorylation of JAK1.

### 5) STC1 Promotes Binding of SOCS1 and JAK1 in THP1 Cells in Presence of IL6 and Promotes Ubiquitination of JAK1 (Immunoprecipitation)

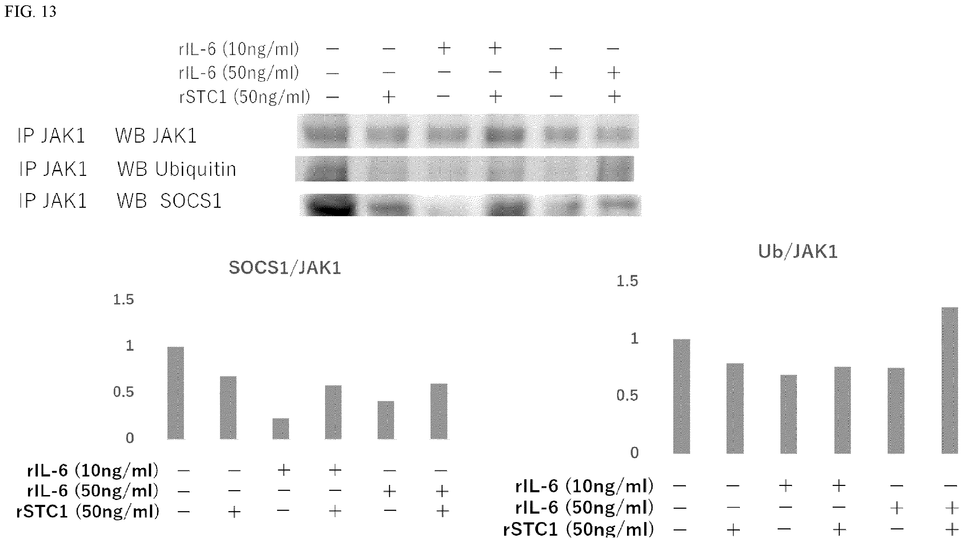

The cell total extracts of the THP1 cells supplemented with IL6 (10 ng/mL), IL6 (50 ng/mL), and/or STC1 (50 ng/mL) as well as the THP1 cells (control) not supplemented with these according to the above method were immunoprecipitated. The results are shown in FIG. 13. For the immunoprecipitation, THP1 whole cell lysate was used, and JAK1 and JAK1-binding proteins were collected using JAK antibody and A/G agarose gel. A reducing agent was added, and the mixture was boiled and then subjected to Western blotting. As shown in FIG. 13, in the presence of IL6, SOCS1 binding to JAK1 decreases, but in the presence of STC1, SOCS1 binding to JAK1 increases. Ubiquitination of JAK1 turned out to be also promoted along with that. That is, STC1 promotes binding of JAK1 and SOCS1 and facilitates ubiquitination of JAK1 (degradation of JAK1).

### 6) STC1 Inhibits Expression of ACE2 and TMPRSS2 Induced by IL6 in THP1 Cells.

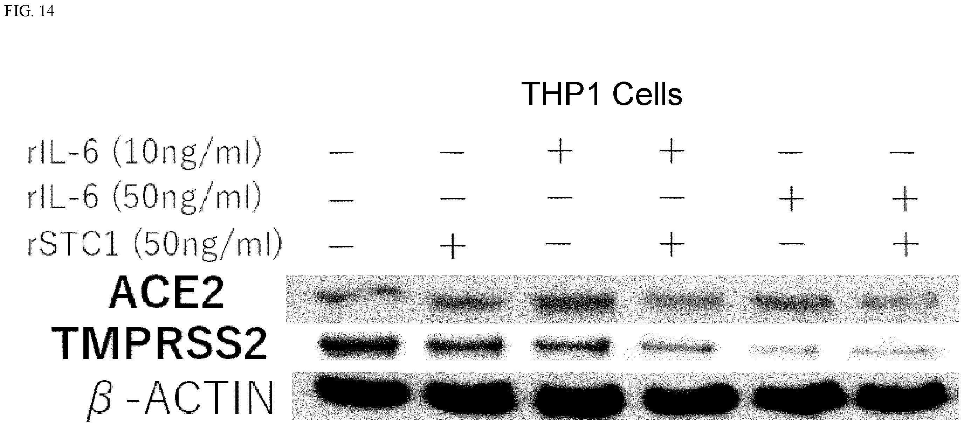

The cell total extracts of the THP1 cells supplemented with IL6 (10 ng/mL), IL6 (50 ng/mL), and/or STC1 (50 ng/mL) as well as the THP1 cells (control) not supplemented with these according to the above method were Western blotted. The results are shown in FIG. 14. As shown in FIG. 14, STC1 inhibited the expression of ACE2 and TMPRSS2. Thus, STC1 inhibits the expression of ACE2 and TMPRSS2, and this may reduce the chance of infection of SARS-CoV2 to cells.

### 7) STC1 Inhibits Expression of ACE2 and TMPRSS2 Dependently on SOCS1.

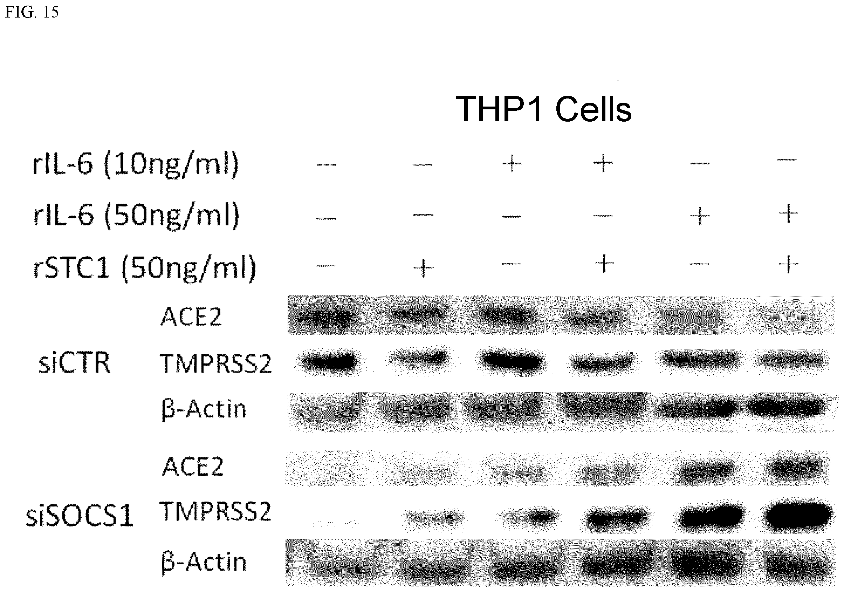

In the cell total extracts of the THP1 cells supplemented with IL6 (10 ng/mL), IL6 (50 ng/mL), and/or STC1 (50 ng/mL) as well as the THP1 cells (control) not supplemented with these according to the above method, SOCS1 was inhibited using siRNA and Western blotted. The results are shown in FIG. 15. siCTR is the control group, and SOCS1 exhibits the same behavior as in the case of being normal. As shown in Fig. 15, the inductive inhibitory effect on ACE2 and TMPRSS2, the effect induced by STC1 in THP1 cells, disappeared when SOCS1 was knocked down using siRNA. This shows that the inhibitory effect on ACE2 and TMPRSS2 by STC1 is SOCS1-dependent.
(Animal model: a mouse bleomycin intratracheal instillation model in a state where inflammation occurs in the lung)

### 8) STC1 Inhibits Expression Induction of JAK1, Phosphorylation of JAK1, and Increase in ACE2 and TMPRSS2 Expression Induced by Bleomycin Intratracheal Instillation in Mouse Lung,

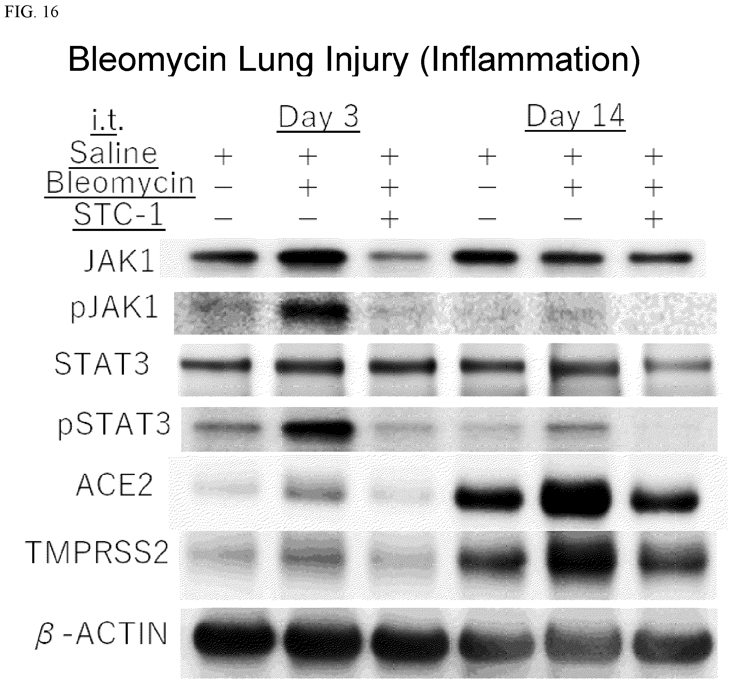

Saline or saline in which bleomycin was dissolved was intratracheally instilled to animals (C57BL6 mice) according to the above method. In the group where the bleomycin-dissolved saline was intratracheally instilled, saline or STC1-dissolved saline was intratracheally instilled next day, and lung tissues were collected after 3 days and 14 days and Western blotted. The results are shown in FIG. 16. As shown in FIG. 16, STC1 inhibited the phosphorylation of JAK1, phosphorylation of STAT3, increase in the expression of ACE2, and increase in the expression of TMPRSS2 due to bleomycin-induced inflammation. Thus, intratracheal instillation of STC1 inhibits the JAK/STAT3 pathway and simultaneously inhibits the expression of ACE2 and TMPRSS2. That is, STC1 inhibits cytokine release syndrome in COVID19 and inhibits the expression of ACE2 and TMPRSS2, and thus may inhibit the infection and aggravation of COVID19.

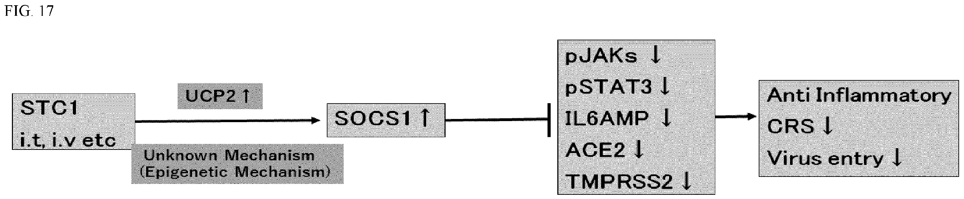

FIG. 17 summarizes the mechanism of action of STC1 elucidated this time.

## Claims

1. A STAT3 phosphorylation inhibitor containing stanniocalcin 1 (STC1) as an active ingredient.

2. A JAK expression or phosphorylation inhibitor containing stanniocalcin 1 as an active ingredient.

3. A prophylactic or therapeutic agent for autoimmune disease, the agent containing stanniocalcin 1 as an active ingredient.

4. A prophylactic or therapeutic agent for influenza virus infection, the agent containing stanniocalcin 1 as an active ingredient.

5. A prophylactic or therapeutic agent for coronavirus infection, the agent containing stanniocalcin 1 as an active ingredient.

6. The prophylactic or therapeutic agent for coronavirus infection according to claim 5, wherein the coronavirus has an S protein capable of binding to ACE2.

7. The prophylactic or therapeutic agent for coronavirus infection according to claim 5 or 6, wherein the coronavirus is a SARS-related coronavirus.

8. An ACE2 and TMPRSS2 expression inhibitor containing stanniocalcin 1 as an active ingredient.

9. An IL6 AMP inhibitor containing stanniocalcin 1 as an active ingredient.

10. The STAT3 phosphorylation inhibitor according to claim 1, the JAK expression or phosphorylation inhibitor according to claim 2, the prophylactic or therapeutic agent for autoimmune disease according to claim 3, the prophylactic or therapeutic agent for influenza virus infection according to claim 4, the prophylactic or therapeutic agent for coronavirus infection according to any one of claims 5 to 7, the ACE2 and TMPRSS2 expression inhibitor according to claim 8, or the IL6 AMP inhibitor according to claim 9, wherein the inhibitor or the agent is an inhalant.
